# EUROPEAN PATENT APPLICATION

(11) **EP 2 055 783 A1**
(43) Date of publication of application: **06.05.2009**
(21) Application number: 07119602.6
(22) Date of filing: 30.10.2007
(51) Int. Cl.: C12N 15/82, C07K 14/81

(54) **Insect inhibiting plant serpin mutants**

(71) Applicant: VIB vzw, 9052 Gent (BE); Universiteit Gent, 9000 Gent (BE)
(72) Inventor: The designation of the inventor has not yet been filed

(57) **Abstract**

The present invention relates to a method to limit insect and/or mite damage in plants, by the use of mutant plant proteinase inhibitors. More specifically, it relates to the use of mutant serpins of which the reactive centre loop has been replaced by an artificial sequence. Preferably, said mutant serpins use an *Arabidopsis thaliana* serpin-1 backbone, or a homologue thereof. Said mutant serpins have a specificity other than the wild type serpins, and by modulating the reactive loop center, specific inhibitors against specific insect and/or mite proteases can be developed. Those mutant serpins can be used to inhibit or limit insect and/or mite damage, such as the damage caused by insect feeding.

SEQUENCE LISTING

<110>  VIB vzw
       Universiteit Gent

<120>  INSECT INHIBITING PLANT SERPIN MUTANTS

<130>  FVB/Serpmut/271

<160>  10

<170>  PatentIn version 3.3

<210>  1
<211>  391
<212>  PRT
<213>  Arabidopsis thaliana

<400>  1

Met Asp Val Arg Glu Ser Ile Ser Leu Gln Asn Gln Val Ser Met Asn
1               5                   10                  15

Leu Ala Lys His Val Ile Thr Thr Val Ser Gln Asn Ser Asn Val Ile
            20                  25                  30

Phe Ser Pro Ala Ser Ile Asn Val Val Leu Ser Ile Ile Ala Ala Gly
        35                  40                  45

Ser Ala Gly Ala Thr Lys Asp Gln Ile Leu Ser Phe Leu Lys Phe Ser
    50                  55                  60

Ser Thr Asp Gln Leu Asn Ser Phe Ser Ser Glu Ile Val Ser Ala Val
65                  70                  75                  80

Leu Ala Asp Gly Ser Ala Asn Gly Gly Pro Lys Leu Ser Val Ala Asn
                85                  90                  95

Gly Ala Trp Ile Asp Lys Ser Leu Ser Phe Lys Pro Ser Phe Lys Gln
            100                 105                 110

Leu Leu Glu Asp Ser Tyr Lys Ala Ala Ser Asn Gln Ala Asp Phe Gln
        115                 120                 125

Ser Lys Ala Val Glu Val Ile Ala Glu Val Asn Ser Trp Ala Glu Lys
    130                 135                 140

Glu Thr Asn Gly Leu Ile Thr Glu Val Leu Pro Glu Gly Ser Ala Asp
145                 150                 155                 160

Ser Met Thr Lys Leu Ile Phe Ala Asn Ala Leu Tyr Phe Lys Gly Thr
                165                 170                 175

Trp Asn Glu Lys Phe Asp Glu Ser Leu Thr Gln Glu Gly Glu Phe His
            180                 185                 190

Leu Leu Asp Gly Asn Lys Val Thr Ala Pro Phe Met Thr Ser Lys Lys
        195                 200                 205

Lys Gln Tyr Val Ser Ala Tyr Asp Gly Phe Lys Val Leu Gly Leu Pro
    210                 215                 220

Tyr Leu Gln Gly Gln Asp Lys Arg Gln Phe Ser Met Tyr Phe Tyr Leu
225                 230                 235                 240

Pro Asp Ala Asn Asn Gly Leu Ser Asp Leu Leu Asp Lys Ile Val Ser
                245                 250                 255

Thr Pro Gly Phe Leu Asp Asn His Ile Pro Arg Arg Gln Val Lys Val
            260                 265                 270

Arg Glu Phe Lys Ile Pro Lys Phe Lys Phe Ser Phe Gly Phe Asp Ala
        275                 280                 285

Ser Asn Val Leu Lys Gly Leu Gly Leu Thr Ser Pro Phe Ser Gly Glu
    290                 295                 300

Glu Gly Leu Thr Glu Met Val Glu Ser Pro Glu Met Gly Lys Asn Leu
305                 310                 315                 320

Cys Val Ser Asn Ile Phe His Lys Ala Cys Ile Glu Val Asn Glu Glu
                325                 330                 335

Gly Thr Glu Ala Ala Ala Ala Ser Ala Gly Val Ile Lys Leu Arg Gly
            340                 345                 350

Leu Leu Met Glu Glu Asp Glu Ile Asp Phe Val Ala Asp His Pro Phe
        355                 360                 365

Leu Leu Val Val Thr Glu Asn Ile Thr Gly Val Val Leu Phe Ile Gly
    370                 375                 380

Gln Val Val Asp Pro Leu His
385                 390

<210>  2
<211>  392
<212>  PRT
<213>  Brassica napus

<400>  2

Met Asp Val Arg Glu Ser Ile Ser Leu Gln Asn His Val Ser Leu Ser

1               5                   10                  15

Leu Ala Lys Asn Val Ile Ser Thr Val Ser Lys Asn Ser Asn Val Ile
            20                  25                  30

Phe Ser Pro Ala Ser Ile Asn Val Val Leu Gly Ile Ile Ala Ala Gly
        35                  40                  45

Ser Thr Gly Glu Thr Lys Asp Gln Ile Leu Ser Phe Leu Asn Phe Pro
    50                  55                  60

Ser Ile Asp Gln Leu Asn Thr Phe Ser Ser Asp Ile Val Ser Ala Val
65                  70                  75                  80

Leu Ala Asp Gly Ser Ala Asn Gly Gly Pro Lys Leu Ser Ala Ala Asn
                85                  90                  95

Ala Pro Trp Ile Asp Lys Ser Leu Ser Leu Gln Pro Ser Phe Lys Gln
            100                 105                 110

Leu Leu Asp Gly Ser Tyr Lys Ala Ala Ser Asn Leu Ala Asp Phe Gln
        115                 120                 125

Thr Lys Ala Val Glu Val Ile Ala Glu Val Asn Ser Trp Ala Glu Lys
    130                 135                 140

Glu Thr Asn Gly Leu Ile Thr Glu Val Leu Pro Glu Gly Ser Ala Asp
145                 150                 155                 160

Ser Met Thr Arg Leu Ile Phe Ala Asn Ala Leu Tyr Phe Lys Gly Thr
                165                 170                 175

Trp Asn Glu Lys Phe Asp Glu Ser Leu Thr Lys Asp Gly Asp Phe His
            180                 185                 190

Leu Leu Asp Gly Ser Ser Lys Val Thr Ala Pro Phe Met Thr Ser Lys
        195                 200                 205

Lys Lys Gln Tyr Val Ser Ala Tyr Asp Gly Phe Lys Val Leu Gly Leu
    210                 215                 220

Pro Tyr Leu Gln Gly Glu Asp Lys Arg Gln Phe Ser Met Tyr Leu Tyr
225                 230                 235                 240

Leu Pro Asp Ala Asn Asn Gly Leu Ser Asp Leu Leu Asp Lys Ile Val
                245                 250                 255

Ser Thr Pro Gly Phe Leu Asp Ser His Ile Pro Arg Arg Gln Val Lys

260                 265                 270

Val Gly Glu Phe Lys Ile Pro Lys Phe Lys Phe Ser Phe Gly Phe Glu
        275                 280                 285

Ala Ser Asp Val Leu Lys Gly Leu Gly Leu Ala Ser Pro Phe Ser Gly
    290                 295                 300

Glu Asp Gly Leu Thr Glu Met Val Glu Ser Pro Glu Met Gly Lys Asn
305                 310                 315                 320

Leu Lys Val Ser Ser Ile Phe His Lys Ala Cys Ile Glu Val Asn Glu
                325                 330                 335

Glu Gly Thr Glu Ala Ala Ala Ala Ser Ala Gly Val Ile Lys Leu Arg
            340                 345                 350

Gly Leu Ala Met Glu Glu Glu Met Ile Asp Phe Val Ala Asp His Pro
        355                 360                 365

Phe Leu Leu Val Val Met Glu Asn Ile Thr Gly Val Ile Leu Phe Ile
    370                 375                 380

Gly Gln Val Ile Asp Pro Leu His
385                 390

<210>  3
<211>  4
<212>  PRT
<213>  Artificial

<220>
<223>  Reactive center loop

<400>  3

Ile Lys Leu Ala
1

<210>  4
<211>  4
<212>  PRT
<213>  Artificial

<220>
<223>  Reactive center loop

<400>  4

Val Arg Pro Arg
1

<210>  5

<211>  4
<212>  PRT
<213>  Artificial

<220>
<223>  Reactive center loop

<400>  5

Ile Lys Leu Lys
1


<210>  6
<211>  17
<212>  DNA
<213>  Artificial

<220>
<223>  Forward primer

<400>  6
atggacgtgc gtgaatc                                                    17


<210>  7
<211>  21
<212>  DNA
<213>  Artificial

<220>
<223>  Reverse primer

<400>  7
ttaatgcaac ggatcaacaa c                                               21


<210>  8
<211>  33
<212>  DNA
<213>  Artificial

<220>
<223>  Reverse mutagenic primer for IKLA

<400>  8
ctccataagc aatcctgcta gctttataac tcc                                  33


<210>  9
<211>  33
<212>  DNA
<213>  Artificial

<220>
<223>  Reverse mutagenic primer for IKLK

<400>  9
ctccataagc aatcctttta gctttataac tcc                                  33


<210>  10
<211>  39
<212>  DNA

<213>  Artificial

<220>
<223>  Reverse mutagenic primer for VRPR

<400>  10
cataagcaat cctcttggcc ttacaactcc agctgatgc                              39

## Description

The present invention relates to a method to limit insect and/or mite damage in plants, by the use of mutant plant proteinase inhibitors. More specifically, it relates to the use of mutant serpins of which the reactive centre loop has been replaced by an artificial sequence. Preferably, said mutant serpins use an *Arabidopsis thaliana* serpin-1 backbone, or a homologue thereof. Said mutant serpins have a specificity other than the wild type serpins, and by modulating the reactive loop center, specific inhibitors against specific insect and/or mite proteases can be developed. Those mutant serpins can be used to inhibit or limit insect and/or mite damage, such as the damage caused by insect feeding.

Insect pests are a serious problem in agriculture. They destroy millions of acres of staple crops such as corn, soybeans, peas, and cotton. Farmers must apply millions of liters of synthetic pesticides to combat these pests. However, synthetic pesticides pose many problems. They are expensive, force the emergence of insecticide-resistant pests, and they constitute a serious risk for the environment.

In order to develop a more economic and environmental pest control, biological approaches to pest control have been tried. In some cases, crop growers have introduced natural predators of the species sought to be controlled, such as non-native insects, fungi, and bacteria like *Bacillus thuringiensis.* Alternatively, crop growers have introduced large colonies of sterile insect pests in the hope that mating between the sterilized insects and fecund wild insects would decrease the insect population. However, none of these methods have been really successful.

As mentioned above, certain species of microorganisms of the genus *Bacillus* are known to possess pesticidal activity against a broad range of insect pests including Lepidoptera (Dulmage *et al.,* 1981), Diptera (Goldberg and Margalit, 1977), Coleoptera (Krieg *et al*., 1987) and Hemiptera (Mathavan *et al.,* 1987). *Bacillus thuringiensis* is probably the most successful biocontrol agents discovered to date. Pesticidal activity appears to be concentrated in parasporal crystalline protein inclusions, although insecticidal proteins have also been isolated from the vegetative growth stage of *Bacillus.* Several genes encoding these insecticidal proteins have been isolated and characterized. As a non limiting example, such genes have been disclosed in W09116432, WO9421795 and US5366892.

Microbial pesticides, particularly those obtained from *Bacillus* strains, have played an important role in agriculture as alternatives to chemical pest control. Insecticidal proteins isolated from strains of *Bacillus thuringiensis,* known as 8-endotoxins or Cry toxins, are initially produced in an inactive protoxin form. These protoxins are proteolytically converted into an active toxin through the action of proteases in the insect gut.

Once activated, the Cry toxin binds with high affinity to receptors on epithelial cells in the insect gut, thereby creating leakage channels in the cell membrane, lysis of the insect gut, and subsequent insect death through starvation and septicemia.

During recent years, agricultural scientists have developed crop plants with enhanced insect resistance by genetically engineering crop plants to produce insecticidal proteins from Bacillus. Such plants have been disclosed in, amongst others, WO9116432 and WO9421795. However, these Bt insecticidal proteins only protect plants from a relatively narrow range of pests. Thus, there is an immediate need for methods that enhance the effects of insecticidal proteins.

One possibility to enhance the insect resistance is the co-expression of the Bt insecticidal proteins with another polypeptide having insecticidal activity, as disclosed in WO2005083095. Alternatively, the application of other broad spectrum insect inhibitory polypeptides, such as proteinase inhibitors has been studied. An overview of proteinase inhibitor genes used in combat against insects, pest and pathogens is given by Haq *et al.* (2004). However, the pH dependency and specificity of the proteinase inhibitor is extremely important and not all proteinases inhibitors are successful. In some cases, the transgenic plants overexpressing the proteinase inhibitor caused a growth stimulation of the larvae (Girard *et al.,* 1998), although the inhibitor used was capable of inhibiting the digestive proteases of the larvae. This indicates that a selection of a proteinase inhibitor purely on in vitro characteristics is very difficult, if not impossible.

Within the group of proteinase inhibitors, plant proteinase inhibitors and within this group, serpins have drawn some interest as possible pest inhibitors. Plant serpins have been studied intensively over the past years. In cereals, serpins are collectively called Z proteins, and constitute 5% of the total albumin in grains (Hejgaard, 1982). Several serpins from wheat, barley, rye, and oat have been identified and characterized (Rasmussen, 1993; Østergaard *et al*., 2000; Brandt *et al*., 1990; Lungaard and Svensson, 1989; Rosenkrands *et al*. 1994; Rasmussen *et al*., 1996; Dahl *et al*., 1996; Hejgaard, 2001; Hejgaard and Hauge, 2002) Biochemical studies with plant serpins from cereals have demonstrated their inhibitory action against different animal proteases such as trypsin, chymotrypsin, cathepsin G, and elastase (Østergaard *et al*., 2000; Dahl *et al*., 1996; Hejgaard, 2001; Hejgaard and Hauge, 2002). Also the serpin isolated from squash phloem was reported to be an elastase inhibitor (Yoo *et al*., 2000)

Whereas in animals, serpins are known to be involved in fundamental biological processes (Patston, 2000; van Gent *et al*., 2003) no precise role has yet been assigned to plant serpins. The high abundance of serpins in cereal seeds has led to the hypothesis that they could be involved in defense of storage tissue against insect feeding (Østergaard *et al*., 2000; Hejgaard and Hauge, 2002). Although direct feeding of aphids with CmPS-1 did not affect their survival, the negative correlation between the survival of aphids feeding on squash leaves and expression levels of the phloem serpin CmPS-1 supports the defense theory (Yoo *et al*., 2000) Gene expression studies in barley have revealed that serpin transcripts are present in developing grain, but also in vegetative tissues, such as roots, shoots, and leaves. Serpin protein could be detected in phloem cells, meristem, and root cap cells of the young root, and in root cap, coleorhiza, and apical meristem of embryonic roots. Also in young leaves some phloem cells produced serpin proteins (Roberts *et al*., 2003). Although these expression patterns are intriguing, complete insight into the precise functional role of plant serpins is still lacking.

Based on this possible protection, several serpins have been tested as insect inhibitors, but none of those have been extremely effective. WO9413810 discloses the use of a *Nicotania alata* type II serine proteinase inhibitor precursor with at least four domains for increasing plant resistance to pest, but the effect is limited to a retardation in growth of the nymphs tested. US2003/0018990 discloses the use of a serine proteinase of *Brassica oleracea* for obtaining resistance to herbivorous insects. Depending upon the parameters measured, and the insects used, the results obtained are comparable or less efficient than the results obtained with Bt. Yoo *et al*. (2000) described the use of *Cucurbita maxima* phloem serpin-1 for inhibiting the piercing-sucking aphid *Myzus persicae*. Although they claim to see a decrease in survival on the transgenic plants in function of the time, they could not demonstrate any toxicity when using the phloem and the effects of survival on the transgenic plants might be due to other factors.

The reactive centre loop (RCL) of serpins is essential for their specificity. The reactive centre loop, as defined here, is defined by the P1 position after which the target proteinase is cleaving the proteinase inhibitor. The RCL as defined here encompasses at least 4 amino acids, including the P1 and aminoterminal of the cleavage site, i.e. P1 is the carboxyterminal amino acid after cleavage. Mutations in the RCL are supposed to block the activity of the serpin, as the RCL acts as substrate for the protease and is exerts its inhibiting activity by competition with the natural substrate. Indeed, Vercammen et al (2006) demonstrated that mutation of the RCL loop into an IKLA or a VRPR sequence resulted in a significant loss of inhibitory activity.

Surprisingly we found that those mutant serpins do have an inhibiting activity against insect proteases, and that this activity may be far pronounced that the activity of the wild type serpin. Mutations of the RCL, without - or with limited - changes in the backbone of the serpin allows to design insect specific serpins, and opens the possibility to design plants with a specific resistance against some insects, without affecting non target organisms.

A first aspect of the invention is the use of a recombinant serpin, comprising a mutant reactive center loop, for limiting insect and/or mite provoked damage in plants. A mutant RCL as used here means that the RCL differs from the sequence that occurs in the corresponding wild type serpin. Preferably, said serpin belongs to the inhibitor family IA, as determined by MEROPS (http://merops.sanger.ac.uk), showing sequence homology to human alpha1-antitrypsin. Preferably said serpin is selected of the group consisting of *Arabidopsis thaliana* serpin At1g47710, Barley serpin BZx, Barley serpin BZ7, Wheat serpin WZ2b, *Cucumis sativus* serpin gi|58416137, *Oryza sativa* serpin gil37700305, *Citrus x paradise* serpin gi|26224736, *Solanum tuberosum* serpin gi|62950609, *Lycopersicon esculentum* serpin gi|14685955, *Nicotiana tabacum* serpin gi|52837819, *Brassica rapa* serpin gi|54416864, *Vitis vinifera* serpin gi|33401535, *Antirrhinus majus* serpin gi|51113970, *Triphysaria versicolor* serpin gi|68033793, *Helianthus paradoxus* serpin gi|33123072, *Gossypium arboretum* serpin gi|13245345, *Populus nigra* serpin gi|60218046 and *Brassica napus* (SEQ ID N° 2). Even more preferably, said the backbone of said serpin is derived from SEQ ID N° 1 (At1g47710). Preferably, said mutant serpin comprises a RCL comprising a sequence selected from the group consisting of IKLA, VRPR and IKLK. Even more preferably, said mutant serpin comprises a RCL comprising IKLA. Preferably, said insect and/or mite caused damage is caused by a chewing insect. More preferably, said insect or mite belongs to an order selected from the group consisting of the orders orders *Lepidoptera, Coleoptera, Homoptera* and *Acari.* Even more preferably, said insect or mite is selected from the group consisting of *Spodoptera* spp, Sesamia spp., *Ostrinia* spp., *Leptinotarsa* spp.,*Tribolium* spp., *Acyrthosiphon* spp., *Bemisia* spp. and *Tetranychus* spp. Most preferably said insects or mites belong to the species *Spodoptera littoralis,* Sesamia *nonagroides, Ostrinia nubilalis, Leptinotarsa decemlineata, Tribolium castaneum, Acyrthosiphon pisum, Bemisia tabaci* and/or *Tetranychus urticae.*

A preferred embodiment is the use of said serpin, whereby said serpin is applied by spraying. Another preferred embodiment is the use of said serpin, whereby said serpin is overexpressed in a plant. In case of overexpressing, the concentration in the plant material is preferably at least 0.65mg/kg wet weight, even more preferably at least 6.5 mg/kg wet weight, most preferably at least 65mg/kg wet weight.

Still another aspect of the invention is a method to develop new mutant serpins with a specific inhibition spectrum, comprising the mutation of one or more amino acids of the RCL. A specific inhibition spectrum as used here means the the mutant serpin inhibits other proteinases than the wild type serpin, or shows a significantly higher inhibition towards a proteinase that is inhibited by the wild type serpin. Significantly higher as used here means that the inhibition occurs at significantly lower concentration of the serpin, and/or that a significantly higher inhibition percentage is reached.

Another aspect of the invention is a transgenic plant, expressing a recombinant serpin, comprising according to the invention. Preferably, said plant is used to limit insect damage caused by insects feeding on the plant.

### BRIEF DESCRIPTION OF THE FIGURES

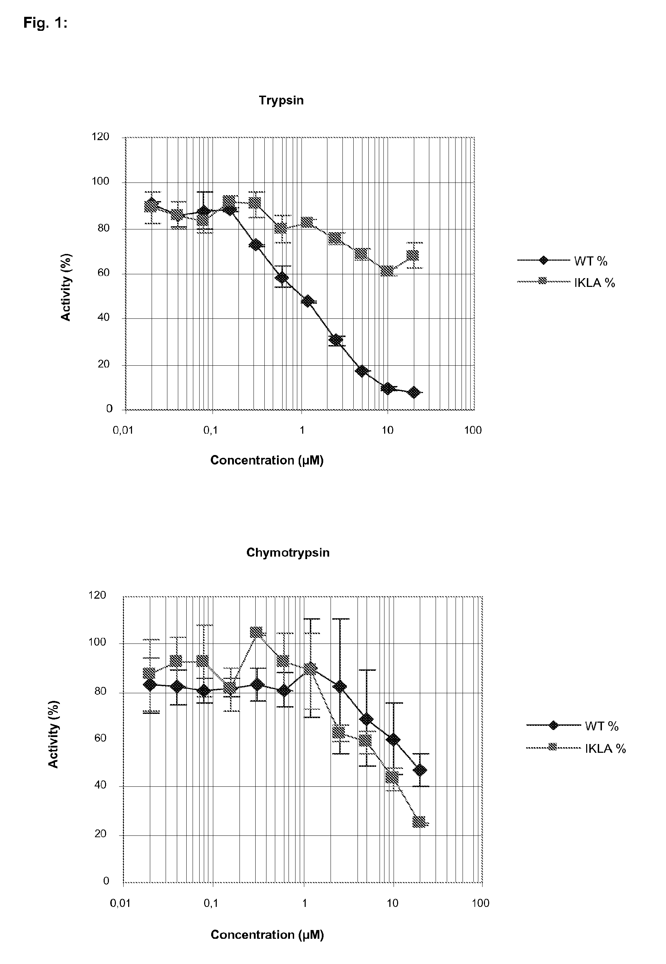

**Figure 1****:** Inhibition of serine-like protease activities from S. *littoralis* extracts by AtSerpin1 (WT) and its derived variant IKLA. Data are the mean ± SE of duplicate measurements from a unique pool of gut extracts.

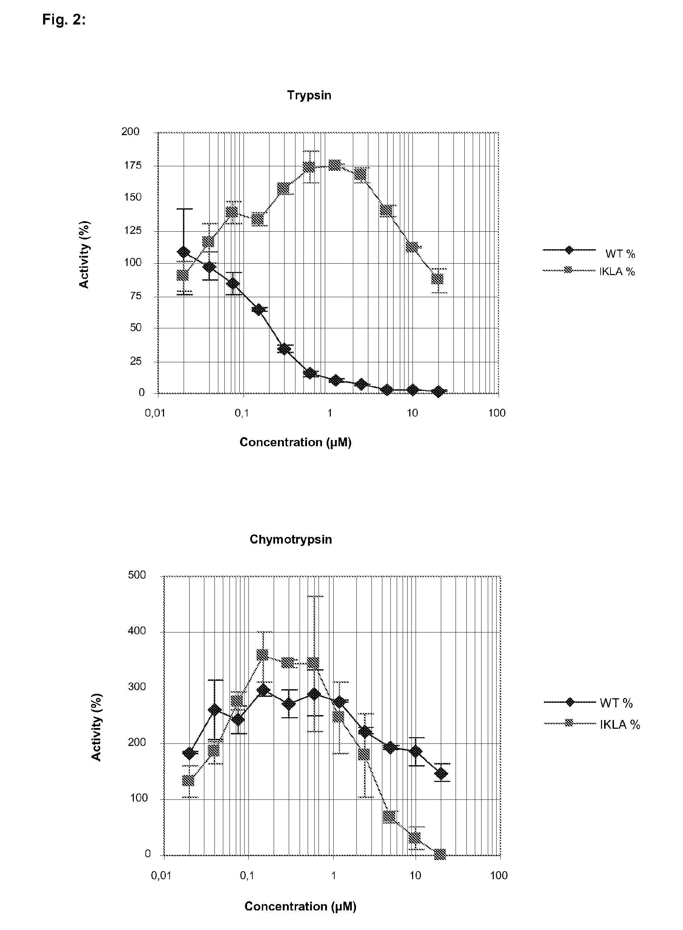

**Figure 2****:** Inhibition of serine-like protease activities from *S. nonagrioides* extracts by AtSerpin1 (WT) and its derived variant IKLA. Data are the mean ± SE of duplicate measurements from a unique pool of gut extracts.

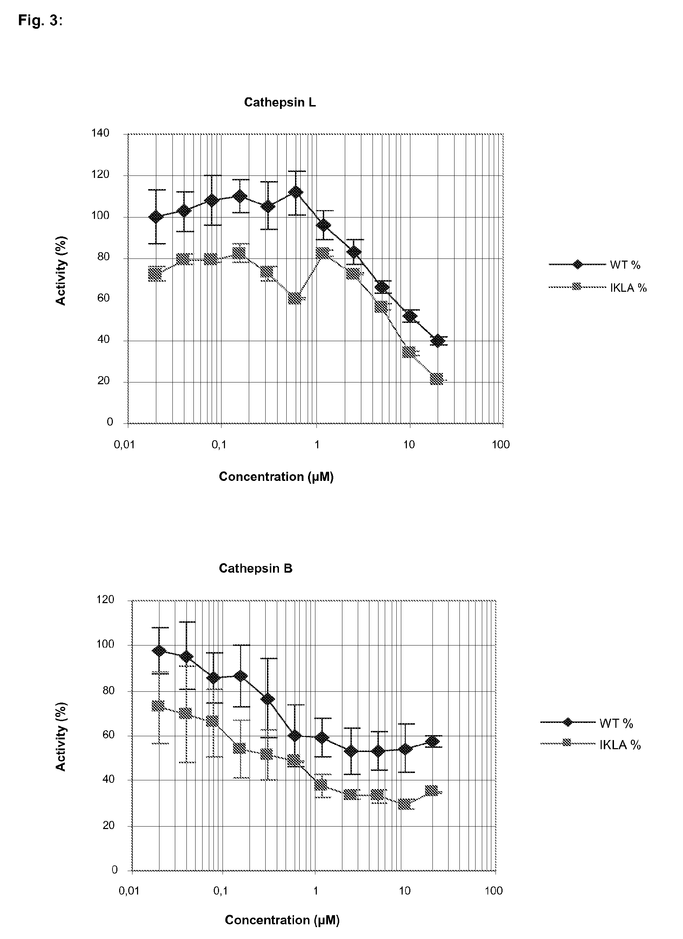

**Figure 3****:** Inhibition of cysteine-like protease activities from *L. decemlineata* extracts by AtSerpin1 (WT) and its derived variant IKLA. Data are the mean ± SE of duplicate measurements from a unique pool of gut extracts.

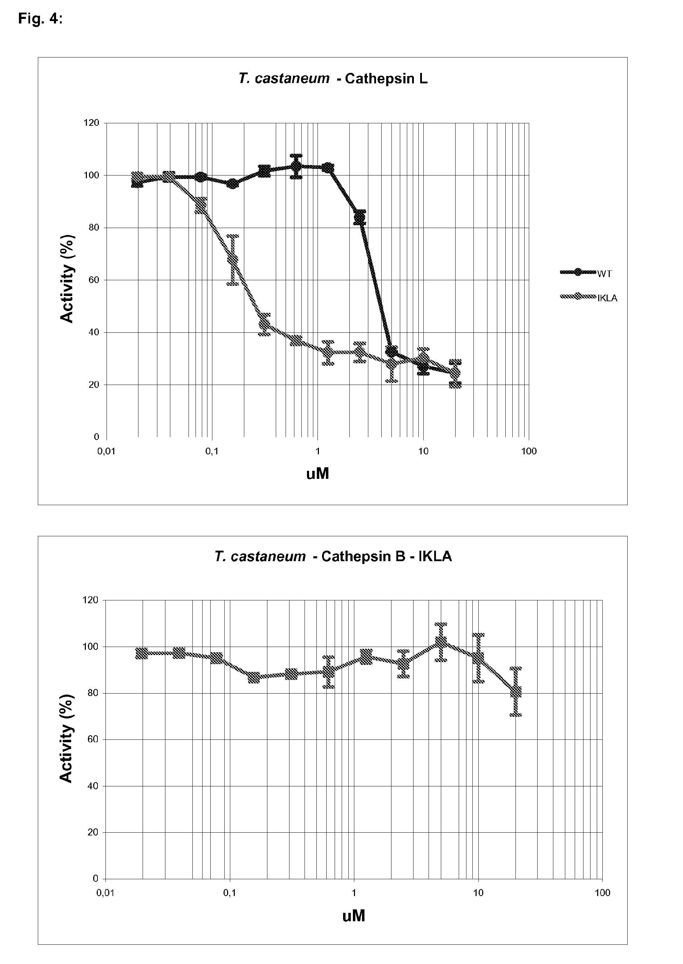

**Figure 4****:** Inhibition of cysteine-like protease activities from *T. castaneum* extracts by AtSerpin1 (WT) and its derived variant IKLA. Data are the mean ± SE of duplicate measurements from a unique pool of gut extracts.

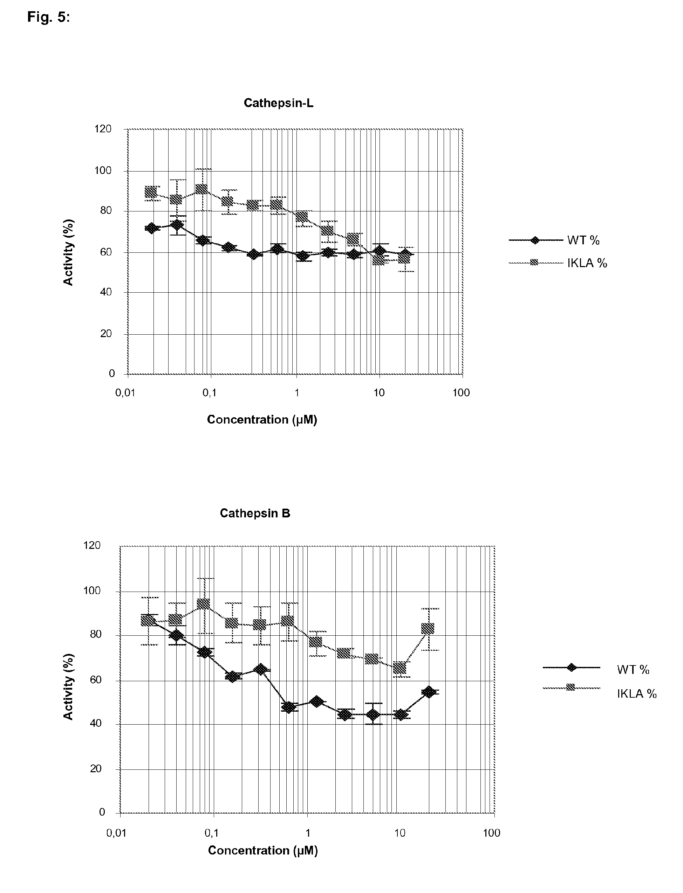

**Figure 5****:** Inhibition of cysteine-like protease activities from *A. pisum* extracts by AtSerpin1 (WT) and its derived variant IKLA. Data are the mean ± SE of duplicate measurements from a unique pool of gut extracts.

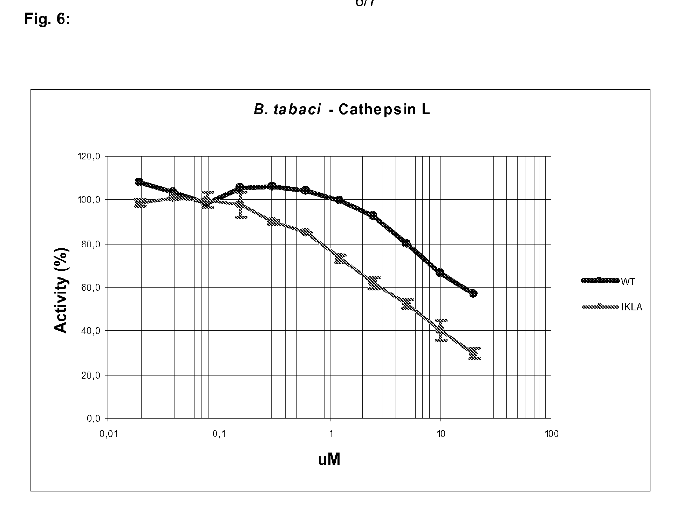

**Figure 6****:** Inhibition of cysteine-like cathepsin-L protease activities from *B*. *tabaci* extracts by AtSerpin1 (WT) and its derived variant IKLA. Data are the mean ± SE of duplicate measurements from a unique pool of gut extracts.

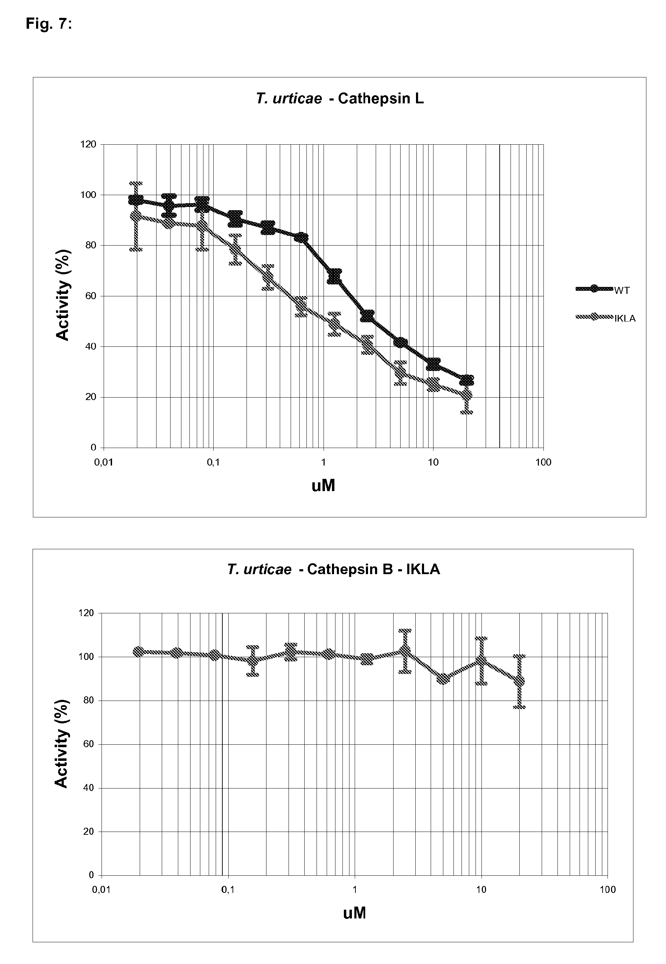

**Figure 7****:** Inhibition of cysteine-like protease activities from T. *urticae* extracts by AtSerpin1 (WT) and its derived variant IKLA. Data are the mean ± SE of duplicate measurements from a unique pool of gut extracts.

### EXAMPLES

### Materials and methods to the examples

### Recombinant Atserpin 1 production; construction and production of the mutants

Recombinant Atserpin1 production and purification was done as described in Vercammen et al., 2006.

The cDNA for the ORF of At1g47710 was obtained by RT-PCR with the following forward and reverse primers, provided with the adequate 5' extensions for Gateway™ cloning (Invitrogen): 5'-ATGGACGTGCGTGAATC-3' and 5'-TTAATGCAACGGATCAACAAC-3'. After recombination in pDEST17, the plasmid was introduced into *E. coli strain* BL21 (DE3)pLysE and production of the HIS6-tagged protein induced by incubation in 0.2 mM isopropyl-β-D-thiogalactopyranoside for 24 h. The protein was purified by metal ion affinity chromatography (TALON™; BD, Franklin Lakes, NJ). Protein concentration and purity were checked by Bradford analysis (BioRAD) and SDS-polyacrylamide gel electrophoresis (PAGE). Point mutagenesis of AtSerpin1 was done using the "megaprimer" method. The reverse mutagenic primers used were: IKLA, 5'- CTCCATAAGCAATCCTGCTAGCTTTATAACTCC-3'; IKLK, 5'- CTCCATAAGCAATCCTTTTAGCTTTATAACTCC-3'; and VRPR, 5'- CATAAGCAATCCTCTTGGCCTTACAACTCCAGCTGATGC-3'. Bacterial production and purification were performed as described for wild-type AtSerpin1.

### Insects

A selection of economically important pest insects was made to support the insecticide potency and wide target pest range of AtSerpins. The selected Lepidoptera (caterpillars) and Coleoptera (beetles) are representative pest insects with biting-chewing mouth paths and important in agriculture, horticulture, forestry and stored products. The selected Homoptera (aphids, whiteflies) and Acari (mites) are good representatives for piercing-sucking pests, being very important in agriculture, horticulture, forestry.

### Lepidoptera:

A colony of the cotton leafworm *Spodoptera littoralis* (CLW) (Lepidoptera: Noctuidae) was reared on a semi-artificial diet at standard conditions in the laboratory. Larvae were collected and frozen at -20°C until analysis.

For the artificial feeding tests, newly moulted (0-6h) 3rd instar larvae of the cotton leafworm *Spodoptera littoralis (Lepidoptera: Noctuidae)* (CLW) were selected from a continuous stock colony in the Laboratory of Agrozoology at Ghent University, Belgium, that was kept at standard conditions of 23±2°C; 65±5% relative humidity and a 16:8 (light:dark) regime (Smagghe et al., 2002).

The cotton leafworm is a polyphagous noctuid species of world economic importance in agriculture and horticulture. Such caterpillars cause high levels of damage in at least 87 crop species belonging to 40 families distributed all over the world. Also many populations developed high levels of resistance towards most insecticide groups (Alford, 2000).

Larvae of Mediterranean corn borer, *Sesamia nonagroides* (MCB) (Lepidoptera: Noctuidae), were frozen and stored at -20°C until needed.

Larvae of European corn borer, *Ostrinia nubilalis* (Lepidoptera: Crambidae) (ECB), were collected and frozen at -20°C until analysis.

### Coleoptera:

Colorado potato beetles *Leptinotarsa decemlineata* (CPB) (Coleoptera: Chrysomelidae) were reared on freshly-cut potato foliage, *Solanum tuberosum,* collected and frozen at -20°C until analysis. Adults of the red flour beetle, *Tribolium castaneum* (Coleoptera: Tenebrionidae) were selected from a colony maintained in the laboratory at standard conditions, frozen and stored at -20°C until needed.

Homoptera: A laboratory colony of the pea aphid *Acyrthosiphon pisum* (PA) (Homoptera: Aphididae) was reared on pea plants, *Vicia faba.* Adults were selected, frozen en stored at -20°C until needed. Green peach aphid adults, *Myzus persicae* (Homoptera: Aphididae) were selected, frozen and stored at -20°C until needed. Adults of the sweetpotato whitefly, *Bemisia tabaci* (Homoptera: Aleyrodidae) were selected, frozen and stored at -20°C until needed.

### Acari:

Spider mites, *Tetranychus urticae* (Acari: Tetranychidae) were selected from a colony maintained in the laboratory at standard conditions, frozen en stored at -20°C until needed.

### Inhibitory activity of Arabidopsis thaliana serpins

Complete guts from CLW, MCB, ECB and CPB larvae were dissected and subsequently homogenized in 0.15 M NaCl, centrifuged at 10,000g for 5 min, and the supernatants pooled and stored frozen (-20°C). For PA, whole insect bodies were used.

Inhibitory activity of wild-type Serpin1 from *Arabidopsis thaliana* and its derived variant IKLA were tested *in vitro* against serine-like protease activities from CLW, MCB (trypsin and chymotrypsin) and ECB (trypsin and elastase), and against cysteine-like protease activities (cathepsin-B and cathepsin-L) from PA and CPB. *A. thaliana* serpins were preincubated at room temperature with the gut extracts for 15 min, prior to addition of substrate. Serpin concentrations varied from 0.02-20 µM. Standard fluorometric substrates used were Z-Arg-Arg-amc, Z-Phe- Arg-amc, and N-Suc-Leu-Leu-Val-Tyr-4-Methylcoumaryl-7-amide, from Bachem. For measuring elastase activity the colorimetric substrate Sa2PppNa (N-succinyl-(alanine)2-prolinephenylalanin-p-nitroanilide) were used, from Sigma. All assays were carried out in duplicate with pooled gut extracts. Fluorescence was monitored for 30 min at λex of 380 nm and λem of 460 nm in a microtiterplate reader. For measuring the elastase activity in ECB, the colorimetric substrate Sa2PppNa (N-succinyl-(alanine)2-proline-phenylalanin-p-nitroanilide) were used, from Sigma.

Absorbance was measured at 410 nm for elastase activity in a microplate reader. Insect protease activities were determined at 30°C, at pH 10.5 for CLW, MCB and ECB, and at pH 7.5 for PA and CPB, in 100 µl of reaction mixture.

Inhibitory activity of the derived variants IKLK and VRPR were also tested in vitro against trypsin activity of CLW and cathepsin B activity of PA. Standard colorimetric substrates used were BApNa (*N*α-benzoyl-DL-arginine-p-nitroanilide) and ZAA2MNA (N-carbobenzoxyalanine-alanine-arginine 4-methoxy-β-naphthyi amide), from Sigma. All assays were carried out in triplicate with pooled gut extracts. Serpin concentrations were 5 and 10 µM. Absorbance was measured at 410 nm for trypsin activity and 520 nm for cathepsin B activity in a microplate reader. Insect protease activities were determined at 30 °C, at pH 10.5 for CLW and at pH 7.5 for PA, in 100 µl of reaction mixture.

### Example 1: inhibition of enzymes of the Cotton leafworm, Spodoptera littoralis (Lepidoptera: Noctuidae)

As shown in Figure 10, AtSerpin1 exerts a clear inhibition of serine-like trypsin activities already at low concentration with 50% inhibition at about 1 µM concentrations. Maximal inhibition of trypsin activity was scored with ≥10 µM. In contrast the IKLA mutant did not show potency to inhibit the trypsin protease activities of S. *littoralis.* Here fluorometric substrate was employed to measure trypsin activities.

For chymotrypsin activity inhibition the concentration of AtSerpin1 needed to inhibit 50% was estimated at 10-20 µM (Figure 10). For the IKLA derived variant of AtSerpin1, its potency to inhibit trypsin activity was lower than the original AtSerpin1, but its activity against chymotrypsin was similar.

With the use of colorimetric substrates, table 1 confirms the high potency of AtSerpin1 to inhibit trypsin protease activities from S. *littoralis* extracts. With the three different derived variants (mutant IKLA, IKLK and VRPR) the activity was not present. The tests for inhibition of chymotrypsin activities with AtSerpin1 and the IKLA mutant showed limited activity with 40-56% inhibition with the highest concentration tested (10 µM).

**Table 1. Inhibition of serine-like protease activities from S. littoralis extracts by the AtSerpin1 and its derived variants.**

| | % Inhibition | | | | | |
|---|---|---|---|---|---|---|
| | Trypsin | | | Chymotrypsin | | |
| | 1.25 µM | 5 µM | 10 µM | 1.25 µM | 5 µM | 10 µM |
| AtSerpin1 | 52 ± 1 | 83 ± 1 | 90 ± 1 | 10 ± 21 | 31 ± 20 | 40 ± 15 |
| Mutant IKLA | 18±1 1 | 32 ± 2 | 40 ± 2 | 11 ± 16 | 41 ± 5 | 56 ± 5 |
| Mutant IKLK | nc | 64 ± 2 | 69 ± 3 | nc | nc | nc |
| Mutant VRPR | nc | 33 ± 1 | 34 ± 3 | nc | nc | nc |

| | | | | | | |
|---|---|---|---|---|---|---|
| nc= not calculated | | | | | | |

### Example 2: inhibition of the enzymes of the Mediterranean corn borer, Sesamia nonagrioides (Lepidoptera: Noctuidae)

Figure 11 demonstrates a very strong inhibition by AtSerpin1 of trypsin activities already at low concentration with 50% inhibition at about 0.1 µM concentrations. Maximal inhibition of trypsin activity was scored already with 0.5-1 µM. The IKLA derived variant scored low inhibitory activity.

For chymotrypsin activity inhibition the concentration of AtSerpin1 needed to inhibit >50% was estimated at ≥10 µM. Interestingly, for the IKLA derived variant of AtSerpin1, its potency to inhibit chymotrypsin activity was higher than the original AtSerpin1.

Table 2 clearly provides confirmation of the high potency of AtSerpin1 to inhibit trypsin protease activities from *S. nonagrioides* extracts. With the derived IKLA variant the activity was lost. The tests for inhibition of chymotrypsin activities with AtSerpin1 showed no activity; however with the mutant IKLA a high activity with 70% inhibition was scored at 10 µM.

**Table 2. Inhibition of serine-like protease activities from S. nonagrioides extracts by the AtSerpin1 and its derived variant IKLA.**

| | % Inhibition | | | | | |
|---|---|---|---|---|---|---|
| | Trypsin | | | Chymotrypsin | | |
| | 1.25 µM | 5 µM | 10 µM | 1.25 µM | 5 µM | 10 µM |
| AtSerpin1 | 90 ± 1 | 97 ± 1 | 98 ± 1 | ni | ni | ni |
| Mutant IKLA | ni | ni | ni | ni | 32 ± 10 | 70 ± 20 |

| | | | | | | |
|---|---|---|---|---|---|---|
| ni= no inhibition | | | | | | |

### Example 3: Inhibition of the enzymes of the European corn borer, Ostrinia nubilalis (Lepidoptera: Crambidae)

With use of colorimetric substrates for trypsin and chymotrypsin activities, AtSerpin1 shows limited potency to inhibit trypsin protease activities from O. *nubilalis* extracts with 28% at 2 µM (Table 3). With the derived IKLA variant the activity was much higher reaching 51% inhibition at 2 µM. The tests for inhibition of chymotrypsin activities with AtSerpin1 showed no activity, whereas the mutant IKLA exerted 21% inhibition at 2 µM.

**Table 3. Inhibition of serine-like protease activities from O. nubilalis extracts by AtSerpin1 and its derived variant IKLA.**

| | % Inhibition | |
|---|---|---|
| | Trypsin | Elastase |
| | 2 µM | 2 µM |
| AtSerpin1 | 28 ± 2 | ni |
| Mutant IKLA | 51 ± 1 | 21 ± 1 |

| | | |
|---|---|---|
| ni= no inhibition | | |

### Example 4: Inhibition of the enzymes of the Colorado potato beetle, Leptinotarsa decemlineata (Coleoptera: Chrysomelidae)

As shown in Figure 12 with use of fluorometric substrates, AtSerpin1 provokes 50% inhibition of cysteine-like cathepsin-L protease activities at ≥10 µM in the Colorado potato beetle gut extracts.

For the cathepsin-B activity inhibition, the concentration of AtSerpin1 needed to inhibit 50% was estimated >10 µM. For the IKLA derived variant of AtSerpin1, its potency to inhibit both cysteine-like protease activities, cathepsin-B and cathepsin-Lactivity, was higher than the original AtSerpin1 (Figure 12).

With use of colorimetric substrates, AtSerpin1 inhibits cathepsin-B protease activities with 40% already at relatively low concentrations of 1.25 µM (Table 4). For cathepsin-L activities the potency of AtSerpin1 for inhibition was lower, yielding 4% at 1.25 and increasing up to 48% at 10 µM. Interestingly with the derived variant IKLA the activity was higher as with the original AtSerpin1 (Table 4). With the lowest concentration tested (1.25 µM) already 62% inhibition of cathepsin-B was scored. The tests for inhibition of cathepsin-L activities with IKLA scored 66% at 10 µM.

**Table 4. Inhibition of cysteine-like protease activities from L. decemlineata extracts by the AtSerpin1 and its derived variant IKLA.**

| | % Inhibition | | | | | |
|---|---|---|---|---|---|---|
| | Cathepsin B | | | Cathepsin L | | |
| | 1.25 µM | 5 µM | 10 µM | 1.25 µM | 5 µM | 10 µM |
| AtSerpin1 | 40 ± 8 | 47 ± 8 | 46 ± 11 | 4 ± 10 | 34 ± 4 | 48 ± 4 |
| Mutant IKLA | 62 ± 5 | 77 ± 3 | 70 ± 2 | 18 ± 2 | 44 ± 2 | 66 ± 1 |

### Example 5: Inhibition of the enzymes of the Red flour beetle, Tribolium castaneum (Coleoptera: Tenebrionidae)

Figure 13 shows that the cysteine-like cathepsin-L activity is inhibited by the AtSerpin1 with an IC50=4 µM. Very interestingly, the IKLA mutant is >10 times more active and the estimated

IC50 yielded 0.2-0.3 uM to inhibit 50% of cathepsin-L activity. For the cathepsin-B activities, no inhibitory activity was scored with IKLA derived mutant of AtSerpin1 when tested up to 20 µM.

### Example 6: inhibition of the enzymes of the Pea aphid, Acyrthosiphon pisum (Homoptera: Aphididae)

As shown in Figure 14, AtSerpin1 exerts inhibition of cysteine-like cathepsin-L protease activities. However, it was strange in this experiment that the effect with AtSerpin1 flattened around 60% inhibition. Otherwise it was clear that the inhibitory activity of AtSerpin1 was stronger than for the IKLA mutant.

For the cysteine-like cathepsin-B activity inhibition of aphid *A. pisum* extracts the concentration of AtSerpin1 needed to inhibit 50% was estimated at around 1 µM (Figure 14). For the IKLA derived variant of AtSerpin1, its potency to inhibit cathepsin-B activity was lower than the original AtSerpin1.

With use of colorimetric substrates, table 4 confirms the high potency of AtSerpin1 to inhibit cathepsin-B protease activities from *A. pisum* aphid extracts (Table 5). 50% inhibition of cathepsin-B was scored with relatively low concentrations of 1.25 µM. For cathepsin-L activities the % of inhibition was somewhat lower yielding 39-42% at 1.25-10 µM.

As shown in table 5, with the derived variant IKLK the activity was similar as with the original AtSerpin1, whereas with IKLA and VRPR the activities were lower. The tests for inhibition of cathepsin-L activities with IKLA scored somewhat similar as AtSerpin1.

**Table 5. Inhibition of cysteine-like protease activities from A. pisum extracts by the AtSerpin1 and its derived variants.**

| | % Inhibition | | | | | |
|---|---|---|---|---|---|---|
| | Cathepsin B | | | Cathepsin L | | |
| | 1.25 µM | 5 µM | 10 µM | 1.25 µM | 5 µM | 10 µM |
| AtSerpin1 | 50 ± 1 | 55 ± 5 | 55 ± 2 | 42 ± 3 | 42 ± 2 | 39 ± 4 |
| Mutant IKLA | 24 ± 6 | 31 ± 1 | 36 ± 3 | 24 ± 6 | 34 ± 4 | 45 ± 1 |
| Mutant IKLK | nc | 49 ± 4 | 66 ± 4 | nc | nc | nc |
| Mutant VRPR | nc | 14 ± 2 | 23 ± 1 | nc | nc | nc |

| | | | | | | |
|---|---|---|---|---|---|---|
| nc= non calculated | | | | | | |

### Example 7: Inhibition of the enzymes of the Sweetpotato whitefly, Bemisia tabaci (Homoptera: Aleyrodidae)

Figure 15 demonstrates that the cysteine-like cathepsin-L activity is inhibited by the AtSerpin1 with the highest concentration tested (20 µM) 45% inhibition was scored. Very interestingly, the IKLA mutant is about 5-10 times more active and the estimated IC50 is around 5 µM to inhibit 50% of cathepsin-L activity.

### Example 8: Spider mites, Tetranychus urticae (Acari: Tetranychidae)

In spider mites, AtSerpin1 is active to inhibit cathepsin-L protease activities with 50% inhibition at about 2 µM (Figure 16). Very interesting, the IKLA derived mutant of AtSerpin1 is even more active with an IC50 <1 µM; which is 2-3 times higher in activity. For the cathepsin-B activities, no inhibitory activity was scored with IKLA derived mutant of AtSerpin1 when tested up to 20 µM.

### REFERENCES

- Alford D.V. (2000). Pest and disease management handbook. British Crop Protection Council. Blackwell Science. Oxford. 615 pp.
- Brandt, A., Svendsen, I. and Hejgaard, J. (1990). A plant serpin gene. Structure, organisation and expression of the gene encoding barley protein Z. Eur. J. Biochem. 194, 499-505.
- Dahl, S., Rasmussen, S.W. and Hejgaard, J. (1996). Heterologous expression of three plant serpins with distinct inhibitory specificities. J. Biol. Chem. 271, 25083-25088.
- Dulmage, H.T. (1981) Production of bacteria for biological control of insects. In "Biological control in crop production", Paparrizas, D.C. ed., pp 129-141.
- Girard, C., Le Metayer, M., Zaccomer, B, Barlet, B., Barlet, E., Williams, I., Bonade-Bottino, M., Pham-Delegue, M.H. and Jouanin, L. (1998). Growth stimulation of beetle larvae reared on a transgenic oilseed rape expressin a cysteine proteinase inhibitor. J. Insect Physiol., 44, 263-270.
- Goldberg, L.J. and Margalit, J. (1977). A bacterial spore demonstrating rapid larvicidal activity against Anopheles segentii, Uranotaenia unguiculata, Culex univittatus, Aedes aegypti and Culex pipiens. Mosquito News, 37, 355-358.
- Hejgaard, J. (1982). Purification and properties of protein Z - a major albumin of barley endosperm. Physiol. Plant, 54, 174-182.
- Hejgaard, J. (2001). Inhibitory serpins from rye grain with glutamine as P1 and P2 residues in the reactive center. FEBS lett. 488, 149-163.
- Hejgaard, J. and Hauge, S. (2002) Serpins of oat (Avena sativa) grain with distinct reactive centres and inhibitory activity. Physiol. Plant 116, 155-163.
- Haq, S.K., Atif, S.M. and Khan, R.H. (2004) Protein inhibitor genes in combat against insects, pest, and pathogens: natural and engineered phytoprotection. Arch. Biochem. Biophys. 431, 145-159.
- Krieg, A., Huger, A.M. and Schnetter, W. (1987). Bacillus thuringiensis var san diego strain M-7 is identical to the formerly in Germany isolated strain BI 256-82, B. thuringiensis subsp. Tenebrionis, which is pathogenic to coleopteran insects. Z. Angew. Ent. 104, 417-425
- Lungard, R. and Svensson, B. (1989). A 39kD barley seed protein of the serpin superfamily inhibits alpha-chymotrypsin. Carlsberg Res. Commun. 54, 173-180.
- Mathavan, S., Velpandi, A., Johnson, J.C. (1987). Sub toxic effects of Bacillus spaericus 1593 M on feeding growth and reproduction of Laccotrephes griseus (Hemiptera: Nepidae). Exp. Biol. 46, 149-153.
- 0stergaard, H., Rasmussen, S.K. Roberts, T.H. and Hejgaard, J. (2000). Inhibitory serpins from wheat grain with reactive centers resembling glutamine-rich repeats of prolamin storage proteins. Cloning and characterization of five major molecular forms. J. Biol. Chem. 275, 33272-33279.
- Patston, P.A. (2000) Serpins and other serine protease inhibitors. Immunol. Today 21, 345-354.
- Rasmussen, S.K. (1993) A gene coding for a new plant serpin. Biochim. Biophys. Acta 1172, 151-154.
- Rasmussen, S.K., Dahl, S.W., Nørgard, A. And Hejgaard, J. (1996). A recombinant wheat serpin with inhibitory activity. Plant Mol. Biol. 30, 673-677.
- Roberts, T.H., Martilla, S., Rasmussen, S.K. and Hejgaard, J. (2003). Differential gene expression for suicide substrate serine proteinase inhibitors (serpins) in vegetative and grain tissues of barley. J. Exp. Bot. 54, 2251-2263.
- Rosenkrands, I. Heygaard, J., Rasmussen, S.K. and Bjørn, S.E. (1994). Serpins from wheat grain, FEBS lett. 343, 75-80.
- Smagghe, G., Decombel, L., Carton, B., Voigt, B., Adam, G. and Tirry, L. (2002). Action of brassinosteroids in the cotton leafworm Spodoptera littoralis. Insect Biochemistry and Molecular Biology 32, 199-204.
- Van Gent, D., Sharp, P., Morgan, K. And Kalsheker, N. (2003). Serpins: structure, function and molecular evolution. Int. J. Biochem. Cell. Biol. 35, 1536-1547.
- Vercammen, D., Belenghi, B., van de Cotte, B., Beunens, T., Gavigan, J.A., De Rycke, R., Brackenier, A., Inzé, D., Harris, J.L. and Van Breusegem, F. (2006). Serpin 1 of Arabidopsis thaliana is a suicide inhibitor for metacaspase 9. J. Mol. Biol.
- Yoo, B.C., Aoki, K., Xiang, Y., Campbell, L.R., Hull, R.J., Xoconostle-Cazares, B., Monzer, J., Lee, J.Y., Ullman, D.E. and Lucas, W.J. (2000). Characterization of Cucurbita maxima Phloem Serpin-1 (CmPS-1). A developmentally regulated elastase inhibitor. J. Biol. Chem. 275, 35122-35128.

## Claims

1. The use of a recombinant serpin, comprising a mutant reactive centre loop for limiting insect and/or mite provoked damage in plants.

2. The use according to claim 1, whereby said mutant serpin is selected of the group consisting of *Arabidopsis thaliana* serpin At1g47710, Barley serpin BSZx, Barley serpin BSZ7, Wheat serpin WSZ2b, *Cucumis sativus* serpin gi|58416137, *Oryza sativa* serpin gi|37700305, *Citrus x paradise* serpin gi|26224736, *Solanum tuberosum* serpin gi|62950609, *Lycopersicon esculentum* serpin gi|14685955, *Nicotiana tabacum* serpin gi|52837819, *Brassica rapa* serpin gi|54416864, *Vitis vinifera* serpin gi|33401535, *Antirrhinus majus* serpin gi|51113970, *Triphysaria versicolor serpin* gi|68033793, *Helianthus paradoxus* serpin g|33123072, *Gossypium arboretum* serpin gi|13245345, *Populus nigra* serpin g|60218046 and *Brassica napus* (SEQ ID N° 2).

3. The use according to claim 2, whereby said mutant serpin is derived from SEQ ID N° 1 (At1g47710)

4. The use of a recombinant serpin, comprising a reactive centre loop comprising a sequence selected from the group consisting of IKLA, VRPR and IKLK for limiting insect and/or mite provoked damage in plants

5. The use according to any of the previous claims, whereby said insect and/or mite provoked damage is caused by a chewing insect.

6. The use according to claim 5, whereby said insect or mite belongs to an order selected from the group consisting of the orders orders Lepidoptera, Coleoptera, Homoptera and Acari.

7. The use according to claim 6, whereby said insect or mite is selected from the group consisting of Spodoptera spp, Sesamia spp., Ostrinia spp., Leptinotarsa spp.,Tribolium spp., Acyrthosiphon spp., Bemisia spp. and Tetranychus spp.

8. The use according to any of the previous claims, where by said mutant serpin is applied by spraying.

9. The use according to claim 1-6, whereby said serpin is overexpressed in a plant.

10. A method to develop new mutant serpins with a specific inhibition spectrum, comprising the mutation of one or more amino acids of the reactive centre loop

11. A transgenic plant, expressing a recombinant serpin, comprising a mutant reactive center loop.

12. A transgenic plant, according to claim 11, whereby said recombinant serpin comprises a reactive center loop comprising a sequence selected from the group consisting of IKLA, VRPR and IKLK.
